# EUROPÄISCHE PATENTSCHRIFT

(11) **EP 1 081 128 B1**
(45) Veröffentlichungstag und Bekanntmachung des Hinweises auf die Patenterteilung: **16.04.2003**
(21) Anmeldenummer: 00117380.6
(22) Anmeldetag: 24.08.2000
(51) Int. Cl.: C07C 209/10

(54) **Verfahren zur Herstellung von Nitrodiphenylaminen**
Process for the preparation of nitrodiphenylamines
Procédé pour la préparation de nitrodiphenylamines

(30) Priorität: 06.09.1999 DE 19942394
(43) Veröffentlichungstag der Anmeldung: 07.03.2001
(73) Patentinhaber: Bayer Aktiengesellschaft, 51368 Leverkusen (DE)
(72) Erfinder: Giera, Henry, Dr., 86862 Grosskitzighofen (DE); Lange, Walter, Dr., 51519 Odenthal (DE); Pohl, Torsten, Dr., Cambridge, MA 02138 (US); Sicheneder, Adolf, Dr., 25551 Hohenlockstedt (DE); Schild, Christoph, Dr., 51373 Leverkusen (DE)

(56) Entgegenhaltungen:
- EP-A- 0 846 676
- DE-A- 19 738 860
- DE-B- 1 518 307
- DE-C- 19 728 760
- US-A- 2 927 943
- US-A- 3 393 241
- US-A- 5 576 460
- JOHN F. HARTWIG ET AL.: "Room-temperature Palladium-catalyzed amination of aryl bromides and chlorides and extended scope of aromatic C-N bond formation with a commercial ligand" JOURNAL OF ORGANIC CHEMISTRY., Bd. 64, Nr. 15, 7. Juli 1999 (1999-07-07), Seiten 5575-5580, XP002153304 EASTON US

## Beschreibung

Die Erfindung betrifft ein Verfahren zur Herstellung von Nitrodiphenylaminen, insbesondere 4-Nitrodiphenylamin (4-NDPA) durch Umsetzung von Nitrohalogenbenzolen mit aromatischen Aminen in Gegenwart eines Palladium-Katalysators und einer Base.

Die Herstellung von Nitrodiphenylaminen durch Umsetzung von entsprechenden aromatischen Aminen mit p-Nitrochlorbenzol in Gegenwart eines Säureakzeptors oder eines Neutralisierungsmittels, gegebenenfalls in Gegenwart eines Katalysators, ist bekannt und beispielsweise beschrieben in DE-A 32 46 151.

Die Nachteile der in der obigen Literatur beschriebenen Verfahren sind oftmals die unzureichenden Selektivitäten, die neben Ausbeuteverlusten in der Regel mehr oder minder aufwendige Reinigungsschritte erforderlich machen, bevor die Nitrodiphenylamine weiter umgesetzt werden können, beispielsweise durch Hydrierung zu 4-Aminodiphenylaminen.

Eine neuere Methode zur Herstellung von Arylaminen durch Umsetzung von Aminen mit aromatischen Verbindungen, beispielsweise auch halogenierte Nitrobenzole, in Gegenwart eines Palladium-Katalysators und einer Base ist in US-5 576 460 beschrieben. Weiterhin ist aus EP-A 846 676 bekannt, beispielsweise Nitrohalogenbenzole mit aromatischen Aminen in Gegenwart eines Palladium-Katalysators, einer Base und eines Halogenids als Co-Katalysator umzusetzen.

In WO 99/01418 wird die Umsetzung in Wasser mit Palladium-Katalysatoren und wasserlöslichen Phosphinen beschrieben. Nachteilig in diesem Verfahren sind die in der Regel geringen Ausbeuten von 20-50 %.

Es war daher wünschenswert, ein Verfahren zur Herstellung von 4-Aminodiphenylaminen zur Verfügung zu stellen, das von aromatischen Aminen ausgeht und durch Umsetzung von entsprechenden p-Nitrohalogenbenzolen in guten Ausbeuten und hohen Reinheiten zu den gewünschten Nitrodiphenylaminen führt.

Gegenstand der vorliegenden Erfindung ist daher ein Verfahren zur Herstellung von Nitrodiphenylaminen durch Umsetzung von Nitrohalogenbenzolen mit aromatischen Aminen in Gegenwart einer Base und eines Palladium-Katalysators, das dadurch gekennzeichnet ist, dass man als Palladium-Katalysator einen Palladium-Phosphin-Komplex oder andere Palladiumkomplexe einsetzt und als Basen Alkali- und/oder Erdalkalimetallcarbonate, -alkoholate und/oder -hydroxide verwendet, wobei man die Basen vor ihrer Verwendung mahlt , so dass die Basen Primärkristallitgrößen von 20 µm und kleiner und spezifische oberflächen von 0,1 bis 10 m²/g aufweisen.

Als Nitrohalogenbenzole werden bevorzugt solche eingesetzt, in denen die Nitrogruppe in para-Stellung zum Halogenrest steht. Als Halogenreste kommen in Frage: Fluor, Chlor, Brom sowie Iod, bevorzugt Chlor und Brom. Selbstverständlich können die Nitrohalogenbenzole noch durch andere Reste substituiert sein, wie beispielsweise C₁-C₄-Alkylreste. Selbstverständlich kann die Nitrogruppe zu den Halogenresten sich auch in einer anderen als der para-Position befinden, wie in 2- und 3-Position.

Als Nitrohalogenbenzole sind beispielsweise zu nennen: 4-Nitro-2-methylchlorbenzol, 4-Nitro-3-methylchlorbenzol, 4-Nitrochlorbenzol, 3-Nitrochlorbenzol, 2-Nitrochlorbenzol. Besonders bevorzugt ist 4-Nitrochlorbenzol.

Als aromatische Amine können in das erfindungsgemäße Verfahren die für eine solche Reaktion bekannten aromatischen Amine eingesetzt werden, beispielsweise Anilin, o-Toluidin, m-Toluidin, p-Toluidin, 4-Ethylanilin, 4-Butylanilin, 4-Isopropylanilin, 3,5-Dimethylanilin sowie 2,4-Dimethylanilin. Bevorzugt ist Anilin. Selbstverständlich können die aromatischen Amine auch in Form von Gemischen, insbesondere isomeren Gemischen, eingesetzt werden.

Bei dem erfindungsgemäßen Verfahren werden im allgemeinen pro Mol Nitrohalogenbenzol 1 bis 10 Mol, bevorzugt 1,5 bis 6 Mol, besonders bevorzugt 2 bis 4 Mol des aromatischen Amins eingesetzt.

Wie zuvor erwähnt, eignen sich für das erfindungsgemäße Verfahren als Katalysatoren insbesondere Palladium-Phosphin-Komplexverbindungen, wobei das Palladium die Wertigkeit 0 oder II besitzt und als Phosphin-Liganden beispielsweise Verbindungen wie Triphenylphosphin, Tri-o-toluylphosphin, Tricyclohexylphosphin, Tri-t-butylphosphin, Bisdiphenylphosphinethan, Bisdiphenylphosphinpropan, Bisdiphenylphosphinobutan, Bisdicyclohexylphosphinoethan, Bisdipehnylphosphinoferrocen, 5,5'-Dichloro-6,6'-dimethoxy-biphenyl-2,2'-diyl-bis-diphenylphosphin, Bis-4,4'-dibenzofuran-3,3'-yl-bisdiphenylphosphin, 1,1'-Bis-diphenylphosphino-diphenylether sowie Bisdiphenylphosphinobinaphthyl in Frage kommen, wobei deren Phenylreste durch Sulfonsäurereste substituiert sein können oder gegebenenfalls durch eine oder mehrere C₁-C₁₂-Alkylgruppen oder C₃-C₁₀-Cycloalkylgruppen ersetzt sein können. Ferner können auch polymergebundene Phosphine als Liganden dienen. Bevorzugt findet Triphenylphosphin als Ligand Verwendung.

Für das erfindungsgemäße Verfahren können aber auch andere als die erwähnten Phosphinliganden eingesetzt werden, wie beispielsweise Stickstoff- oder Sauerstoffhaltige Liganden bzw. auch zwei oder mehrere verschiedene Heteroatome enthaltene Liganden.

Als Palladium-Verbindungen sind beispielsweise folgende Verbindungen zu nennen: Pd₂dba₃, Pd(acac)₂, Pd(OAc)₂, PdCl₂, (CH₃CN)₂Pd(NO₂)Cl, aber auch andere Palladiumhalogenide, wie Bromide und Iodide, -acetate, -carbonate, -ketonate, -nitrate, -acetonate oder Palladacyclen sein. Ferner sind auch heterogene oder immobilisierte Palladiumkatalysatoren im erfindungsgemäßen Verfahren einsetzbar. Dabei kann ein bereits vorher präparierter Komplex eingesetzt oder ein Komplex in situ aus einer geeigneten Pd-Verbindung und einem Liganden gebildet werden.

Bei den erfindungsgemäß einzusetzenden Palladium-Phosphin-Komplexen beträgt das Verhältnis des entsprechenden Liganden zu Palladium etwa 40:1 bis 1:1, bevorzugt 10:1 bis 2:1 und besonders bevorzugt 8:1 bis 4:1.

Erfindungsgemäß werden die Palladium-Phosphin-Komplexe oder die anderen Komplexe im allgemeinen in Mengen von 0,0001 Mol-% bis 10 Mol-%, bevorzugt 0,001 Mol-% bis 5 Mol-%, bezogen auf die eingesetzten Nitrohalogenbenzolen, eingesetzt.

Als Basen werden im erfindungsgemäßen Verfahren Alkali- und/oder Erdalkalimetallcarbonate, -alkoholate und/oder -hydroxide eingesetzt, wobei insbesondere Kalium- und/oder Natriumcarbonat, Cäsiumcarbonat, Natriummethanolat und Bariumhydroxid zu nennen sind. Bevorzugt werden Kalium und/oder Natriumcarbonat eingesetzt. Die Basen können in unterstöchiometrischer Menge oder im Überschuss bis zur zehnfachen äquivalenten Menge bezüglich des Nitrohalogenbenzols eingesetzt werden. Besonders bevorzugt werden die Basen in Mengen von 0,3 bis 2 Äquivalenten bezogen auf Nitrohalogenbenzol eingesetzt.

Das erfindungsgemäße Verfahren kann bei Temperaturen im Bereich von 20 bis 250°C, bevorzugt jedoch bei Temperaturen von 120 bis 180°C durchgeführt werden. Die Reaktionstemperaturen hängen dabei insbesondere von der Art der Ausgangsprodukte, des Katalysators und der eingesetzten Basen ab.

Das erfindungsgemäße Verfahren kann sowohl in Anwesenheit als auch in Abwesenheit eines geeigneten Lösungsmittels durchgeführt werden. Als Lösungsmittel kommen beispielsweise inerte, organische Kohlenwasserstoffe, wie Xylol und Toluol, in Frage. Weiterhin können die eingesetzten aromatischen Amine selbst als Lösungsmittel fungieren.

In dem beschriebenen Verfahren kann wahlweise analog zu DE-A 26 33 811 und DE-A 32 46 151 das entstehende Reaktionswasser beispielsweise durch Zuhilfenahme eines geeigneten Schleppmittels durch Destillation entfernt werden bzw. auch darauf verzichtet werden.

Die Menge der eingesetzten Lösungsmittel kann leicht durch entsprechende Vorversuche bestimmt werden. Für das erfindungsgemäße Verfahren ist es wesentlich, dass die eingesetzten Basen durch Mahlung und/oder Trocknung vorbehandelt werden.

Die Mahlung kann im erfindungsgemäßen Verfahren beispielsweise in handelsüblichen Küchen- bzw. Kugelmühlen erfolgen. Die Maßnahme des Mahlens bewirkt hierbei eine drastische Vergrößerung der spezifischen Oberfläche, die zu einer deutlichen Steigerung des Umsatzes führt. In vielen Fällen ist durch die Mahlung eine Vergrößerung der spezifischen Oberfläche um den Faktor 10 bis 20 zu beobachten. Im beschriebenen Verfahren wird dadurch unter vergleichbaren Reaktionsbedingungen eine Steigerung der Umsätze von ca. 5 bis 45 % auf 85 bis 100 % erreicht. Die Vergrößerung der spezifischen Oberfläche kann dabei durch Mahlen, Fällen oder durch andere geeignete Maßnahmen erzielt werden.

Im erfindungsgemäßen Verfahren sind zum Erreichen hoherer Umsätze eine Vergrößerung der spezifischen Oberfläche auf 0,1 bis 10 m²/g, bevorzugt 0,2 bis 1 m²/g erforderlich.

Untersuchungen zur Morphologie belegen, dass zur Steigerung der Umsätze durch Mahlung der Basen Primärkristallitgrößen von 20 µm und kleiner erzielt werden müssen.

Aufgrund der ausgeprägten hygroskopischen Eigenschaften der im erfindungsgemäßen Verfahren eingesetzten Basen, neigen diese zur mehr minder starken Aufnahme atmosphärischer Bestandteile, wie Wasser und Kohlendioxid. Ab einer Aufnahme von 30 Gewichtsprozent ist hierbei ein deutlicher Einfluss auf die zu erreichenden Umsätze feststellbar.

Die Trocknung der Basen erfolgt dabei beispielsweise derart, dass unter vermindertem Druck von 0,01 bis 100 mbar für mehrere Stunden auf Temperaturen zwischen 50 und 200°C, bevorzugt zwischen 100 und 160°C erhitzt wird.

Die Durchführung des erfindungsgemäßen Verfahrens kann nach üblichen Methoden in kontinuierlicher oder diskontinuierlicher Weise erfolgen.

Nach dem erfindungsgemäßen Verfahren werden die entsprechenden Nitrodiphenylaminc mit hohen Selektivitäten (>98 %) und je nach eingesetzter Katalysatormenge in Ausbeuten bis zu 99 % erhalten. Die nach dem erfindungsgemäßen Verfahren hergestellten Nitrodiphenylamine werden in besonders hohen Reinheiten erhalten. Weitere Vorteile gegenüber den bekannten Verfahren sind die wesentlich verkürzten Reaktionszeiten von 10 Minuten bis etwa 2 Stunden zu nennen, so dass sich mit dem erfindungsgemäßen Verfahren sehr hohe Raum-Zeit-Ausbeuten erzielen lassen.

Die nach dem erfindungsgemäßen Verfahren hergestellten Nitrodiphenylamine können beispielsweise durch eine Hydrierungsreaktion in die entsprechenden Aminodiphenylamine übergeführt werden, beispielsweise kann durch die Hydrierung von 4-Nitrodiphenylamin 4-Aminodiphenylamin (4-ADPA) erhalten werden, welches ein wichtiges Zwischenprodukt für die Herstellung von Alterungsschutzmittel und für Stabilisatoren in der Gummi- und Polymerindustrie darstellt.

### Beispiele

### Beispiel 1

Vorbehandlung der Basen:

Beispielsweise wird kommerziell verfügbares Kaliumkarbonat für ca. 5 Minuten in einer Küchen- bzw. Kugelmühle gemahlen. Das auf diese Weise behandelte Kaliumcarbonat der Firma Grüssing erfährt hierdurch eine Vergrößerung der spezifischen Oberfläche von 0,04 m²/g auf 0,52 m²/g und eine Primärkristallitgröße von 10 µm und kleiner. Anschließend wird das gemahlene Kaliumcarbonat für 5 Stunden bei einem Druck von 1 mbar und einer Temperatur von 150°C getrocknet Bei Verwendung anderer Basen werden diese in analoger Weise vorbehandelt.

### Beispiel 2

In einem Dreihals-Rundkolben werden 186 g (2 mol) Anilin, 152,5 mg (0,5 mmol) Palladiumacetonylacetonat und 524 mg (2 mmol) Triphenylphosphin unter Argon vorgelegt und 10 Minuten bei Raumtemperatur gerührt. Man gibt 78,5 g (0,5 mol) 4-Chlornitrobenzol zu und rührt für weitere 10 Minuten bei Raumtemperatur. Sodann werden 96,6 g (0,7 mol) Kaliumcarbonat zugegeben, welches zuvor unter der in Beispiel 1 beschriebenen Weise vorbehandelt wurde. Unter starkem Rühren wird für eine Stunde auf 170°C erhitzt, wobei die gaschromatographische Untersuchung einer Probe nach 0,5 Stunden Reaktionszeit schon vollständigen Umsatz belegt.

Man lässt auf 70°C abkühlen, verdünnt mit 750 ml Essigsäureethylester und wäscht mit 500 ml Wasser. Die organische Phase wird mit einer Lösung aus 100 g Natriumchlorid und 500 ml Wasser extrahiert und nach Trennung der Phasen Anilin und Wasser bei 70°C durch Destillation entfernt.

Man erhält 107 g (100 % der Theorie) eines bräunlichen Feststoffes, der laut GC-Analytik eine Reinheit größer 99 % aufweist.

### Beispiel 3

In einem Dreihals-Rundkolben werden 3,750 g (40 mmol) Anilin, 3,1 mg (0,00336 mmol) Palladiumdibenylidenaceton und 7,0 mg (0,027 mmol) Triphenylphosphin unter Argon vorgelegt und 10 Minuten bei Raumtemperatur gerührt. Man gibt 1,055 g (6,72 mmol) 4-Chlornitrobenzol zu und rührt für weitere 10 Minuten bei Raumtemperatur. Sodann werden 1,298 g (9,4 mmol) Kaliumcarbonat zugegeben, welches nicht zuvor unter der in Beispiel 1 beschriebenen Weise vorbehandelt wurde. Unter starkem Rühren wird für eine Stunde auf 170°C erhitzt. Nach gaschromatographische Untersuchung wird ein Umsatz von 48 % erreicht.

### Beispiel 4

In einem Dreihals-Rundkolben werden 3,750 g (40 mmol) Anilin, 3,1 mg (0,00336 mmol) Palladiumdibenylidenaceton und 7,0 mg (0,027 mmol) Triphenylphosphin unter Argon vorgelegt und 10 Minuten bei Raumtemperatur gerührt. Man gibt 1,055 g (6,72 mmol) 4-Chlornitrobenzol zu und rührt für weitere 10 Minuten bei Raumtemperatur. Sodann werden 1,298 g (9,4 mmol) Kaliumcarbonat zugegeben, welches zuvor unter der in Beispiel 1 beschriebenen Weise vorbehandelt wurde. Unter starkem Rühren wird für eine Stunde auf 170°C erhitzt. Nach gaschromatographische Untersuchung wird einUmsatz von 97 % erreicht.

### Beispiel 5

In einem Dreihals-Rundkolben werden 186 g (2 mol) Anilin, 152,5 mg (0,5 mmol) Palladiumacetonylacetonat und 524 mg (2 mmol) Triphenylphosphin unter Argon vorgelegt und 10 Minuten bei Raumtemperatur gerührt. Man gibt 78,5 g (0,5 mol) 4-Chlornitrobenzol zu und rührt für weitere 10 Minuten bei Raumtemperatur. Sodann werden 48,3 g (0,35 mol) Kaliumcarbonat zugegeben, welches zuvor unter der in Beispiel 1 beschriebenen Weise vorbehandelt wurde. Unter starkem Rühren wird für eine Stunde auf 170°C erhitzt, wobei die gaschromatographische Untersuchung einer Probe nach 0,5 Stunden Reaktionszeit schon vollständigen Umsatz belegt.

Man lässt auf 70°C abkühlen, verdünnt mit 500 ml Essigsäureethylester und wäscht mit 500 ml Wasser. Die organische Phase wird mit einer Lösung aus 100 g Natriumchlorid und 500 ml Wasser extrahiert und nach Trennung der Phasen Anilin und Wasser bei 70°C durch Destillation entfernt.

Man erhält 106,55 g (99 % der Theorie) eines bräunlichen Feststoffes, der laut GC-Analytik eine Reinheit von 99 % aufweist.

### Beispiel 6

In einem Dreihals-Rundkolben werden 186 g (2 mol) Anilin, 152,5 mg (0,5 mmol) Palladiumacetonylacetonat und 623 mg (1 mmol) 2,2'-Bis-(diphenylphosphino)-1,1'binaphthyl unter Argon vorgelegt und 10 Minuten bei Raumtemperatur gerührt. Man gibt 78,5 g (0,5 mol) 4-Chlornitrobenzol zu und rührt für weitere 10 Minuten bei Raumtemperatur. Sodann werden 96,6 g (0,7 mol) Kaliumcarbonat zugegeben, welches zuvor unter der in Beispiel 1 beschriebenen Weise vorbehandelt wurde. Unter starkem Rühren wird für eine Stunde auf 170°C erhitzt, wobei die gaschromatographische Untersuchung einer Probe nach 0,5 Stunden Reaktionszeit schon vollständigen Umsatz belegt.

### Beispiel 7

In einem Dreihals-Rundkolben werden 18,6 g (0,2 mol) Anilin, 15,3 mg (0,05 mmol) Palladiumacetonylacetonat und 53,8 mg (0,1 mmol) Bis-(2-(diphenylphosphino)-phenyl)-ether unter Argon vorgelegt und 10 Minuten bei Raumtemperatur gerührt. Man gibt 7,85 g (0,05 mol) 4-Chlornitrobenzol zu und rührt für weitere 10 Minuten bei Raumtemperatur. Sodann werden 9,66 g (0,07 mol) Kaliumcarbonat zugegeben, welches zuvor unter der in Beispiel 1 beschriebenen Weise vorbehandelt wurde. Unter starkem Rühren wird für eine Stunde auf 170°C erhitzt, wobei die gaschromatographische Untersuchung einer Probe nach 0,5 Stunden Reaktionszeit 99 %igen Umsatz belegt.

### Beispiel 8

In einem Dreihals-Rundkolben werden 9,376g (0,1 mol) Anilin, 7,6 mg (0,0083 mmol) Palladiumdibenylidenaceton und 17,6 mg (0,067 mmol) Triphenylphosphin unter Argon vorgelegt und 10 Minuten bei Raumtemperatur gerührt. Man gibt 2,64 g (0,0168 mol) 4-Chlornitrobenzol zu und rührt für weitere 10 Minuten bei Raumtemperatur. Sodann werden 2,502 g (0,0236 mol) Natriumcarbonat zugegeben, welches zuvor unter der in Beispiel 1 beschriebenen Weise vorbehandelt wurde. Unter starkem Rühren wird für eine Stunde auf 175°C erhitzt. Nach gaschromatographische Untersuchung wird ein Umsatz von 74 % erreicht.

## Patentansprüche

1. Verfahren zur Herstellung von Nitrodiphenylaminen durch Umsetzung von Nitrohalogenbenzolen mit aromatischen Aminen in Gegenwart einer Base und eines Palladium-Katalysators, **dadurch gekennzeichnet, dass** man als Palladium-Katalysator einen Palladium-Phosphin-Komplex oder andere Palladiumkomplexe einsetzt und als Basen Alkali- und/oder Erdalkalimetallcarboxylate, -alkoholate und/oder -hydroxide verwendet, wobei man die Basen vor ihrer Verwendung mahlt, so dass die Basen Primärkristallitgrößen von 20 µm und kleiner und spezifische Oberflächen von 0,1 bis 10 m²/g aufweisen.

## Claims

1. Process for the preparation of nitrodiphenylamines by reaction of nitrohalogenobenzenes with aromatic amines in the presence of a base and a palladium catalyst, **characterized in that** a palladium-phosphine complex or other palladium complexes are employed as the palladium catalyst and alkali metal and/or alkaline earth metal carboxylates, alcoholates and/or hydroxides are used as bases, the bases being ground before their use, so that the bases have primary crystallite sizes of 20 µm and less and specific surface areas of 0.1 to 10 m²/g.

## Revendications

1. Procédé de préparation de nitrodiphénylamines par réaction de nitrohalogénobenzènes avec des amines aromatiques en présence d'une base et d'un catalyseur au palladium, **caractérisé en ce que** l'on met en oeuvre comme catalyseur au palladium, un complexe palladium-phosphine ou un autre complexe du palladium et on utilise comme base, un carbonate, alcoolate et/ou hydroxyde de métal alcalin et/ou alcalino-terreux, où l'on broye les bases avant leur utilisation, de sorte que les bases présentent des tailles de cristallites primaires de 20 µm et moins, et des surfaces spécifiques de 0,1 à 10 m²/g.
